# DEMANDE DE BREVET EUROPEEN

(11) **EP 3 354 724 A1**
(43) Date de publication de la demande: **01.08.2018**
(21) Numéro de dépôt: 17305082.4
(22) Date de dépôt: 25.01.2017
(51) Int. Cl.: C12N 5/074

(54) **PROCEDE DE REPROGRAMMATION DE CELLULES SOMATIQUES DE RUMINANTS**

(71) Demandeur: Institut National De La Recherche Agronomique, 75007 Paris (FR); ALLICE, 75012 Paris (FR); INSTITUT NATIONAL DE LA SANTE ET DE LA RECHERCHE MEDICALE (INSERM), 75013 Paris (FR)
(72) Inventeur: PAIN, Bertrand, 63830 NOHANENT (FR); BAQUERRE, Camille, 69008 LYON (FR); JEAN, Christian, 69500 BRON (FR)
(74) Mandataire: Regimbeau

(57) **Abrégé**

La présente invention concerne un procédé de préparation *in vitro* de cellules souches à partir de cellules somatiques de ruminants, par l'expression exogène des gènes CDX-2 et c-MYC dans lesdites cellules somatiques. L'invention concerne également les cellules souches ainsi obtenues, ainsi que leur utilisation pour l'expansion d'un fond génétique individuel, du fait de l'amplification du patrimoine génétique des cellules somatiques initiales, pour le criblage de molécules anti-prolifératrices et le test de l'action de molécules anti-prolifératives, et pour répliquer et tester la virulence de certains pathogènes. Ces cellules souches présentent certaines caractéristiques des cellules souches pluripotentes, telles que l'auto-renouvellement, une prolifération infinie, la détection d'une activité télomérase, un cycle cellulaire spécifique et l'expression de marqueurs particuliers.

## Description

### DOMAINE DE L'INVENTION

La présente invention concerne un procédé de préparation *in vitro* de cellules souches à partir de cellules somatiques de ruminants, par l'expression exogène des gènes CDX2 et c-MYC dans lesdites cellules somatiques. L'invention concerne également les cellules souches ainsi obtenues, ainsi que leur utilisation pour l'expansion d'un fond génétique individuel, du fait de l'amplification du patrimoine génétique des cellules somatiques initiales, pour le criblage de molécules anti-prolifératrices et le test de l'action de molécules anti-prolifératives, et pour répliquer et tester la virulence de certains pathogènes. Ces cellules souches présentent certaines caractéristiques des cellules souches pluripotentes, telles que l'auto-renouvellement, une prolifération infinie, la détection d'une activité télomérase, un cycle cellulaire spécifique et l'expression de marqueurs particuliers.

### ETAT DE LA TECHNIQUE

Les ruminants et en particulier les bovins représentent une grande partie de la production animale en France, en Europe et dans le monde. Le cycle de vie des animaux étant particulièrement long, le développement de toute méthode permettant de maintenir à long terme et d'amplifier le potentiel génétique d'un animal, via une cellule avec un potentiel germinal en particulier, est un objectif prioritaire pour les filières.

Les cellules souches et les cellules souches embryonnaires (ES) en particulier présentent les caractéristiques uniques d'auto-renouvellement et de différenciation *in vitro* et *in vivo* dans tous les lignages embryonnaires. Pour les seules cellules ES, il y a colonisation du lignage germinal avec la propriété de produire des descendants avec le même génotype que celui des cellules quand elles sont injectées dans un embryon receveur. Si des cellules ES ont été isolées dans plusieurs espèces, cette définition stricte de colonisation germinale est actuellement restreinte aux seuls rongeurs. On peut qualifier les autres types cellulaires dits ES de cellules 'ES-like' en l'absence de cette propriété fondamentale, notamment pour les espèces de mammifères autres que les rongeurs et les espèces non mammifères. Ces cellules 'ES-like' ont été isolées, amplifiées et établies en lignées avec des propriétés d'auto-renouvellement et de différenciation dans de nombreuses espèces parmi lesquelles on peut citer sans être exhaustif, l'homme, les primates non humains, les espèces de rente comme les ruminants, les ovins, caprins, équins, lapins, les chiens et chats, les oiseaux, comme le poulet et le canard, les poissons comme le médaka, le poisson zèbre (zebrafish), le turbo....etc... La plupart de ces cellules ont été caractérisées par leur potentiel de prolifération, de différenciation *in vitro* et par la présence de certains marqueurs comme des antigènes de surface dont SSEA1, SSEA3 et SSEA4, antigènes identifiés initialement chez la souris, mais dont la réactivité croisée avec d'autres espèces s'est avérée très importante pour identifier ces cellules (Koh et Piedrahita, 2014). Parmi ces cellules, très peu présentent la propriété de colonisation de l'embryon, même au niveau somatique avec les exceptions notables des cellules de poulet (Pain et al., 1996) et de poisson zèbre (zebrafish) (Hong et al., 2004).

Si les cellules ES-like sont actuellement identifiées dans de nombreuses espèces de mammifères, ces cellules sont encore particulièrement difficiles à obtenir à partir des embryons et à maintenir dans un état non différencié dans des cultures à long terme. Beaucoup d'espèces restent en fait plus ou moins réfractaires à la dérivation en routine de ces cellules (porcins, ovins, ruminants, chiens, chats, lapins, etc ...) et sont inaccessibles pour des génotypes spécifiques ou souhaités de par la rareté et le nombre limité d'embryons généralement disponibles dans ces espèces (Koh et Piedrahita, 2014).

Au cours de ces dix dernières années, la reprogrammation somatique est une des découvertes parmi les plus prometteuses dans le domaine des cellules souches. En 2006, le professeur S.Yamanaka démontrait pour la première fois que l'introduction d'un cocktail de gènes dans une cellule différenciée (un fibroblaste) changeait le devenir de la cellule en lui redonnant les propriétés similaires à celles des cellules souches embryonnaires (ES). Ce processus de reprogrammation était obtenu par la combinaison de quatre facteurs de transcription, les produits des gènes Oct4, Sox2, Klf4 et c-MYC (OSKM), encore appelée combinaison de Yamanaka. Par la suite, diverses combinaisons de gènes ont été également identifiées comme le cocktail OSNL (Oct4, Sox2, Nanog et Lin28), appelée combinaison de Thomson (Yu et al., 2007). D'autres acteurs, y compris Nr5a2 (Heng et al., 2009), ESRRB (Feng et al., 2009), ZIC3 (Declercq et al., 2013), TBX3 (Han et al., 2010), miR302, (Anokye-Danso et al., 2011) et d'autres participent directement au processus de reprogrammation ou augmentent l'efficacité de reprogrammation (Hochedlinger & Plath, 2009, Stadtfeld & Hochedlinger, 2010). Mais dans tous les cas, il n'y a pas de substitution majeure dans la combinaison d'origine.

Plus récemment, un processus de reprogrammation somatique appelé reprogrammation directe a également été développé, et qui fait appel à l'utilisation d'une combinatoire de gènes spécifiques d'un lignage cellulaire défini pour obtenir, à partir d'une cellule somatique, une cellule du lignage exprimant les facteurs de reprogrammation. Il a été ainsi possible avec une combinaison originale de différents facteurs de transcription de générer des cardiomyocytes fonctionnels avec la combinaison Gata4, MEF2C, Tbx5 (Idea et al., 2010), avec Ascl1, Brn2, Mytl1 pour produire des neurones (Vierbuchen et al., 2010), avec Sox10, Olig2, ZPF536 pour produire des précurseurs d'oligodendrocytes (Yang et al., 2013) et avec la combinaison FOXA3, HNF1A et HNF4A pour produire des hépatocytes (Yu et al., 2013, Huang et al., 2014). Il s'agit donc par ce processus d'obtenir des cellules d'un lignage donné à partir de l'introduction dans une cellule somatique différenciée (comme un fibroblaste) d'une combinaison de facteurs de transcription spécifiques du lignage identifié.

D'abord démontré dans le modèle de la souris, l'approche de reprogrammation a ensuite été utilisée avec succès sur des fibroblastes humains, sur le rat, sur des cellules de primates non humains et avec plus ou moins de succès dans un grand nombre d'espèces de mammifères, y compris le lapin (Honda et al, 2013; Osteil et al, 2013), les ovins et les bovins. (Liu et al, 2012; Sumer et al, 2011), le porc (Ezashi et al, 2009), le chien (Shimada et al, 2010) et même dans certaines espèces en voie de disparition (Verma et al, 2013). Il est parfois très difficile de vérifier dans les publications si les gènes exogènes ne s'expriment plus et si les cellules sont donc bien reprogrammées. En particulier la stabilité génétique des cellules présentées n'est pas illustrée et la question de leur établissement dans la durée demeure ouverte avec l'absence de courbe de croissance. Chez les espèces non mammifères, les résultats sont plus rares avec seulement quelques rapports controversés pour les cellules aviaires, (Lu et al, 2012 ; Rosselo et al, 2013) et des tests sur d'autres espèces (Rosselo al., 2013).

Au niveau des ovins et bovins, Sumer et al., 2011 ont décrit l'obtention de cellules souche pluripotentes induites ou « iPS » par modification de la combinaison classique de gènes OSKM à laquelle le gène NANOG a été ajouté. Les auteurs démontrent une meilleure efficacité avec cette addition et l'expression de marqueurs de pluripotence comme l'alcaline phosphatase SSEA1 et SSEA4. Les auteurs mentionnent l'obtention de cellules ou clones établis, sans que ne soit précisé le nombre de jours ou de passages au-delà de 10 et 12. La seule combinaison OSKM, sans NANOG, ne permet d'obtenir des cellules que sur 7 à 9 passages. Dans les deux cas, aucune courbe de croissance n'illustre le propos et il n'y a pas d'établissement à long terme.

Par conséquent, les tentatives de reprogrammation ont toujours été réalisées avec les facteurs OSKM et/ou avec la variante associant les gènes NANOG et LIN28 dérivés de la combinatoire de Thomson. Au regard de la littérature, il semble difficile d'obtenir des cellules bovines qui présentent effectivement les caractéristiques des cellules souches ou *« ES-like* », notamment sur des critères de prolifération, de marqueurs et de potentiel de différenciation *in vitro* dans différents lignages embryonnaires.

### RESUME DE L'INVENTION

La présente invention est relative à un procédé de préparation *in vitro* de cellules souches, comprenant la culture de cellules somatiques de ruminants et l'expression exogène des gènes CDX2 et c-MYC dans lesdites cellules somatiques.

Le gène CDX2 (caudal-type homeobox 2) est un gène homéotique de type caudal membre de la famille des facteurs de transcription qui code une protéine régulatrice majeure des gènes spécifiquement impliqués dans la différenciation et la croissance cellulaire de l'intestin. Cette protéine joue un rôle dans le développement embryonnaire précoce du tractus intestinal.

L'invention est également relative aux cellules souches de ruminants telles qu'obtenues par le procédé ci-dessus (ci-après dénommées cellules BCM), et à leur utilisation pour l'expansion d'un fond génétique individuel, du fait de l'amplification du patrimoine génétique des cellules somatiques initiales, pour le criblage de molécules anti-prolifératrices et le test de l'action de molécules anti-prolifératives, et pour répliquer et tester la virulence de pathogènes.

De préférence, et outre les gènes exogènes exprimés, ces cellules expriment la télomérase (TERT) et notamment les gènes suivants: GH1, LRP2, UGT8, JAG2, BRCA2, NBEA, CAMK2B, DSP, AMPH, SCIN, IGSF11, SLC6A15, CECR2, et WNK2.

La présente invention est aussi relative à l'utilisation de l'expression exogène des gènes CDX-2 et c-MYC dans des cellules somatiques de ruminants pour préparer des cellules souches et à diverses utilisations des cellules souches de ruminants obtenues.

### DESCRIPTION DETAILLEE DE L'INVENTION

La présente invention est relative à un procédé de préparation *in vitro* de cellules souches, comprenant la culture de cellules somatiques de ruminants et l'expression exogène des gènes CDX2 et c-MYC dans lesdites cellules somatiques.

L'invention consiste à utiliser le gène CDX2 comme acteur principal de reprogrammation en association avec c-MYC, en présence ou en l'absence de gènes de pluripotence, tels que OCT4 et/ou SOX2 notamment, et de gènes germinaux, tels que DAZL, DDX4, NANOS et PIWI en particulier. Pris indépendamment, ni CDX2 ni c-MYC ne produisent d'effet sur les différents types de fibroblastes embryonnaires testés (issus de différents ruminants), mais en association, les cellules BEF (*Bovine Embryonic Fibroblast*)*,* GEF (*Goat Embryonic Fibroblast*)*,* et SEF (*Sheep Embryonic Fibroblast)* montrent un changement drastique de morphologie quelques jours après l'expression de la combinatoire dans les cellules. Les cellules obtenues présentent alors une morphologie et une cinétique de prolifération similaires à celles de cellules souches embryonnaires.

Dans le cadre de l'invention, les gènes mentionnés sont les suivants :

**Tableau 1 : * pour les gènes CDX2, c-MYC, OCT4, SOX2, KLF4 d'origine humaine et les gènes NANOG et TERT d'origine bovine, identifiés selon la base NCBI ( https://www.ncbi.nlm.nih.gov/ ; rubrique de recherche GENE)**

| **Gene *** | **Gene ID** | **Synonyme** | **Nom du gène** | Version |
|---|---|---|---|---|
| CDX2 | 1045 | CDX3, CDX2/AS | Caudal type Homeobox 2 | 21/12/2016 |
| c-MYC | 4609 | MRTL; MYCC; c-Myc; bHLHe39 | v-myc avian myelocytomatosis viral oncogene homolog | 01/01/2017 |
| POU5F1- OCT4 | 5460 | OCT3; OCT4; OTF3; OTF4; OTF-3; Oct-3; Oct-4 | POU class 5 homeobox 1 | 25/12/2016 |
| SOX2 | 6657 | ANOP3; MCOPS3 | SRY-box 2 | 25/12/2016 |
| KLF4 | 9314 | EZF; GKLF | Kruppel like factor 4 | 25/12/2016 |
| NANOG | 538951 | | Nanog homeobox | 31/10/2016 |
| TERT | 518884 | | telomerase reverse transcriptase | 31/10/2016 |

Selon un mode de réalisation préféré, le procédé de préparation de cellules selon l'invention comprend en outre l'expression exogène d'au moins un des gènes choisis dans le groupe constitué de OCT4, SOX2 et KLF4, de préférence au moins OCT4, dans lesdites cellules somatiques.

L'invention permet ainsi d'obtenir des cellules souches de ruminants par reprogrammation de cellules somatiques de ruminants. Les cellules qui résultent de cette reprogrammation sont uniques dans leur caractérisation. Elles présentent des propriétés similaires aux cellules souches embryonnaires comme un auto-renouvellement, une prolifération rapide avec un temps de doublement moyen compris entre 10 et 13 heures contre 60 h environ pour les fibroblastes avant reprogrammation sans phase de sénescence, un cycle cellulaire typique d'une cellule souche avec une phase G0/G1 courte par rapport à celle observée pour les fibroblastes, une expression et une activité de la télomérase et l'expression d'un programme moléculaire unique.

Par « ruminant », on entend un groupe de mammifères onguligrade qui présentent la particularité de ruminer, à savoir la faculté de remastiquer leur aliment (essentiellement de l'herbe et des végétaux) après les avoir ingérer une première fois. On distingue trois grandes familles de ruminants, les Bovidés, famille qui réunit les ruminants à cornes creuses et la plupart des ruminants domestiques (boeuf, buffle, chèvre, mouton, etc...), les Girafidés, à cornes courtes et pleines et les Elaphidés ou Cervidés, famille qui regroupe notamment les ruminants dont les cornes tombent annuellement quand elles ont fini leur croissance (cerf, daim, renne, etc ...). Une des particularités physiologique de ce groupe est la complexité de leur estomac constitué de quatre poches distinctes permettant le processus de rumination.

Le procédé selon l'invention a comme « produit d'origine », ou cellules de départ ou cellules initiales, des cellules somatiques de ruminants. Par « cellules somatiques », on entend des cellules d'un lignage donné, autres que des cellules souches, des cellules souches pluripotentes et des cellules germinales.

Parmi les cellules somatiques qui peuvent être utilisées dans le procédé selon l'invention, on peut notamment citer les fibroblastes, les cellules musculaires, les cellules blanches du sang circulant telles que monocytes, macrophages, lymphocytes, etc... , les adipocytes, hépatocytes, les cellules endothéliales, etc...

Les cellules somatiques utilisées peuvent être de n'importe quelle espèce de ruminants, en particulier elles peuvent être des cellules somatiques bovines, caprines ou ovines.

Par définition, les cellules somatiques n'expriment pas le gène de la télomérase ni les gènes de pluripotence tels que le marqueur NANOG (Chambers and Tomlinson, 2009).

Par conséquent, le procédé de préparation de cellules souches selon l'invention utilise des cellules somatiques qui n'expriment ni le gène de la télomérase ni le gène NANOG.

Dans le cadre du procédé de l'invention, tout type de système de délivrance des gènes peut être utilisé pour conduire l'expression exogène de CDX2 et c-MYC. Comme exemples de système de délivrance on peut néanmoins citer les stratégies intégratives, notamment celles utilisant des constructions rétrovirales par exemple, ou bien les stratégies non-intégratives faisant appel par exemple à la transfection directe d'ARN, de plasmides d'expression tels que des plasmides épisomaux, à l'infection adénovirale, au virus de Sendai, etc. Des moyens efficaces ont également été développés avec des cassettes polycistroniques d'expression des transgènes. Des systèmes exempts de virus et basés sur des vecteurs transposons floxés permettant l'excision ultérieure des transgènes ont été développés (Woltjen et al., 2009; Kaji et al., 2009). Tous ces développements visent à minimiser l'effet néfaste et non contrôlé de l'intégration d'un transgène sur l'intégrité du génome et sur la stabilité à long terme des cellules reprogrammées. Le choix de produire une cellule génétiquement altérée (GA) qui ne présente qu'une cicatrice mineure de sa modification est une avancée par rapport à la production d'une cellule génétiquement modifiée (GM) qui conserve la modification de façon définitive.

De manière avantageuse, le système de délivrance des gènes, qu'il soit de type intégratif ou non-intégratif, est un système inductible, qui peut par conséquent être contrôlé à loisirs. Ainsi, selon un mode de réalisation particulier, l'expression des gènes exogènes dans le procédé de préparation de cellules selon l'invention est une expression inductible. Par exemple, l'expression des gènes peut être conduite par des vecteurs inductibles dont l'expression peut être contrôlée par la présente d'un composé inducteur tel que la doxycycline par exemple. Ainsi, selon un mode préféré du procédé de l'invention, l'expression des gènes CDX2 et c-MYC est une expression inductible.

Dans le cadre de la présente invention, les gènes exprimés dans les cellules somatiques de ruminants peuvent être issus d'une origine quelconque en termes d'espèces, et notamment d'une espèce différente des ruminants. De préférence les gènes exogènes exprimés peuvent être d'origine humaine, ou bien de ruminants, en particulier d'origine bovine.

Les cellules somatiques sont mises en culture dans un milieu approprié. Le « milieu approprié » désigne notamment un milieu de culture classique, adapté aux cellules somatiques de ruminants, et le plus souvent liquide. L'homme du métier spécialiste en culture cellulaire saura déterminer le ou les milieux appropriés pour faire croître les cellules *in vitro,* dans des conditions optimisées, notamment en termes d'équilibre acide base du milieu de culture, de température et de concentration contrôlée en CO₂.

Dans le cadre de l'invention, lorsque le système d'expression choisi est un système d'expression inductible, l'expression inductible est de préférence réalisée de manière transitoire. Dans ce cas, l'agent inducteur est de préférence « enlevé » de la culture par diminution progressive, amenant à une diminution progressive de l'expression des gènes exogènes CDX-2 et c-MYC, et le cas échéant du ou des autres gènes exogènes tels que OCT4, SOX2 et KLF4. Par exemple dans le cas d'une induction par la doxycycline, la concentration ajoutée au milieu est progressivement diminuée pour aboutir à son absence complète.

Les différentes étapes de culture des cellules somatiques et d'expression exogènes de CDX-2 et c-MYC sont pratiquées pendant le temps nécessaire. En particulier, l'homme du métier suivra le changement morphologique et la reprogrammation des cellules en visualisant, à l'aide d'un microscope, leur croissance et leur viabilité, et sera à même de décider si l'étape doit se poursuivre au-delà du temps minimum indiqué.

L'étape de culture des cellules somatiques est réalisée de préférence pendant une trentaine de jours, au maximum une cinquantaine de jours. Dans tous les cas, avant entrée en sénescence des cellules..

L'étape d'expression exogènes des gènes CDX-2 et c-MYC est réalisée de préférence sur une période d'au moins 10 jours, avantageusement de 15 à 30 jours, période permettant de stabiliser le phénotype des cellules obtenues. Après cette période, l'induction de l'expression des gènes exogènes CDX-2 et c-MYC, et le cas échéant du ou des autres gènes exogènes tels que OCT4, SOX2 et KLF4 peut progressivement être diminuée.

Selon un mode de réalisation préférée, le procédé de préparation *in vitro* de cellules souches selon l'invention comprend, outre l'expression exogènes de CDX-2 et c-MYC, l'expression d'au moins un des gènes choisis dans le groupe constitué de OCT4, SOX2 et KLF4, de préférence au moins OCT4, dans lesdites cellules somatiques.

La reprogrammation des somatiques par l'expression exogène de CDX-2 et c-MYC se traduit par un changement morphologique drastique, l'aspect allongée d'une grande cellule fibroblastique avec un cytoplasme abondant des cellules initiales disparaissant au profit d'une petite cellule ronde très réfringente dans laquelle le rapport nucléocytoplasmique est très élevé avec un cytoplasme très limité, et par l'acquisition de marqueurs et de propriétés cellulaires comparables aux cellules souches, en particulier en termes d'auto-renouvellement, de prolifération, de cycle cellulaire, d'expression et d'activité de la télomérase.

Le produit résultant du procédé consiste en des cellules souches de ruminants qui peuvent être maintenues avec le même phénotype, sans phase de senescence, de vieillissement ou d'épuisement, pendant au moins 20 passages, de préférence au moins 30 passages, plus préférentiellement au moins 50 passages, ce qui correspond environ à au moins 80 générations de préférence plus de 100 à 120 générations dans un milieu de culture approprié. Ces cellules souches de ruminants expriment la télomérase et l'activité correspondante, présentent un cycle cellulaire avec une phase G0/G1 très courte, proliférent rapidement et n'exprimant pas les marqueurs OCT4, SOX2, NANOG de pluripotence caractéristiques des cellules souches embryonnaires.

Selon un second aspect, la présente invention a donc pour objet les cellules souches de ruminants susceptibles d'être obtenues par le procédé de reprogrammation tel que défini ci-dessus. Les cellules souches de ruminants selon l'invention sont ici dénommées cellules BCM pour (Bovine CDX2 c-MYC) du fait de l'expression exogène d'au moins CDX2 et c-MYC.

Par « cellule souche pluripotente », on entend la propriété d'une cellule à se différencier *in vitro* et *in vivo* dans tous les types cellulaires d'un embryon et d'un organisme (Niwa, 2007). Les états de pluripotence naïf et induit ont été caractérisés plus récemment en fonction de l'origine des cellules et de leur condition de maintien en culture *in vitro* (Nichols and Smith, 2009). L'ensemble de ces cellules présentent des caractéristiques moléculaires, épigénétiques spécifiques ainsi que des propriétés développementales particulières, notamment la capacité de colonisation de la lignée germinale pour les cellules pluripotentes naïves (De Los Angeles et al., 2015). Parmi les critères retenus pour caractériser une cellule souche pluripotente, on peut citer l'auto-renouvellement des cellules, l'expression de facteurs de transcription comme OCT4, SOX2, NANOG, des niveaux de méthylation de l'ADN souvent hypométhylé, une activité télomérase, un profil de cycle cellulaire particulier avec une phase G0/G1 courte par rapport à celle d'une cellule somatique.

Les cellules obtenues selon l'invention sont de petites cellules rondes ou ovoïdes adhérentes, mais qui peuvent aussi proliférer en suspension sans support quand elles sont cultivées en boites non traitées pour la culture. Elles n'expriment pas les marqueurs de pluripotence OCT4, SOX2, KLF4 et NANOG tels que définis dans la littérature selon le consensus actuel. Ces cellules sont donc des cellules souches de ruminants et non des cellules souches pluripotentes ; elles peuvent donc également être définies comme des cellules souches non pluripotentes de ruminants.

Outre la combinatoire de gènes exogènes qui est exprimée dans les cellules somatiques pour obtenir les cellules souches de l'invention, à savoir l'expression exogènes de CDX-2 et c-MYC éventuellement associée à l'expression d'au moins un des gènes choisis dans le groupe constitué de OCT4, SOX2 et KLF4, ces cellules expriment en particulier la télomérase TERT, et l'un au moins des gènes suivants : GH1, LRP2, UGT8, JAG2, BRCA2, NBEA, CAMK2B, DSP, AMPH, SCIN, IGSF11, SLC6A15, CECR2, WNK2. Plus préférentiellement, elles expriment au moins deux, au moins trois, au moins quatre, au moins cinq, au moins six, au moins sept, au moins huit, au moins neuf, au moins dix, au moins onze, au moins douze, au moins treize ou quatorze de ces gènes. Encore plus préférentiellement, elles expriment l'ensemble de ces quatorze gènes.

Par ailleurs, les cellules souches de l'invention n'expriment pas les gènes endogènes de pluripotence suivants : OCT4, SOX2 et NANOG. En revanche, elles expriment les protéines exogènes qui correspondent à la combinatoire de gènes utilisée : au moins CDX2 et c-MYC, et éventuellement OCT4, SOX2, KLF4.

Dans le cadre de l'invention, les gènes mentionnés sont les suivants :

**Tableau 2 : selon la base NCBI (https://www.ncbi.nlm.nih.gov/ ;rubrique de recherche GENE**

| **Gene *** | **Gene ID** | **NCBI SeqRef** | **Version** | **Nom du gène** |
|---|---|---|---|---|
| CDX2 | 618679 | NM_001206299.1 | 24-DEC-2015 | caudal type homeobox 2 |
| GH1 | 280804 | NM_180996.1 | 24-AVR-2016 | growth hormone 1 |
| LRP2 | 100337021 | XM_015462263.1 | 26-JAN-2016 | LDL receptor related protein 2 |
| UGT8 | 281566 | NM_001083635.1 | 24-AVR-2016 | UDP glycosyltransferase 8 |
| JAG2 | 517405 | XM_015468638.1 | 26-JAN-2016 | jagged 2, transcript variant X1 |
| BRCA2 | 507069 | XM_002684277.4 | 26-JAN-2016 | breast cancer 2, transcript variant X1 |
| NBEA | 525624 | XM_015465645.1 | 26-JAN-2016 | neurobeachin, transcript variant X5 |
| CAMK2B | 525416 | NM_001035357.2 | 29-JUIL-2016 | calcium/calmodulin-dependent protein kinase II beta |
| DSP | 514360 | NM_001192368.1 | 04-JAN-2015 | desmoplakin |
| AMPH | 614722 | NM_001098130.1 | 24-AVR-2016 | amphiphysin |
| SCIN | 281478 | NM_174177.2 | 24-AVR-2016 | scinderin |
| IGSF11 | 540003 | NM_001076921.1 | 25-AOUT-2016 | immunoglobulin superfamily member 11 |
| SLC6A15 | 281952 | NM_181023.2 | 25- AOUT-2016 | solute carrier family 6 (neutral amino acid transporter), member 15 |
| CECR2 | 517202 | XM_015463582.1 | 26-JAN-2016 | cat eye syndrome chromosome region, candidate 2 |
| WNK2 | 506520 | XM_005199274.3 | 26-JAN-2016 | WNK lysine deficient protein kinase 2, transcript variant X3 |

Les cellules souches de l'invention présentent une activité télomérase. Ces cellules expriment le gène TERT dont le niveau d'expression du transcrit est corrélé à l'activité de la télomérase. Cette activité est détectable dans des conditions similaires aux cellules ES de souris, prises comme référence de cellules souches et connues pour exprimer une activité importante de télomérase ; activité qui est absente dans les fibroblastes somatiques initiaux.

La capacité de prolifération des cellules souches de l'invention est infinie telle que testée avec une prolifération observées pendant au moins 150 jours consécutifs sans modification de leur vitesse de prolifération quand elles sont maintenues dans des conditions identiques de culture et sans changement notable de phénotype.

Selon un aspect préféré de l'invention, les cellules souches obtenues ont un phénotype stable pendant au moins 20 passages, préférentiellement au moins 30 passages, plus préférentiellement au moins 50 passages, à partir de leur établissement. Par établissement, on entend l'obtention de cellules qui se propagent de façon pérenne avec une vitesse de prolifération soutenue, régulière et constante sur une période de temps importante, au-delà de la phase d'entrée en sénescence des cellules somatiques, évaluée à 100 jours environ pour les fibroblastes bovins.

Le terme « passage » désigne la mise en culture de cellules ayant atteint un taux d'occupation maximum de leur support. Les cellules sont détachées de leur support par action rapide d'une enzyme ou d'un cocktail d'enzymes protéasiques (trypsine, pronase, dispase, collagénase) dissociant les cellules entre elles et favorisant leur détachement de la matrice extracellulaire (action de « trypsination ») et sont diluées dans du milieu de culture avant d'être ensemencées sur un nouveau support pour une nouvelle mise en culture de quelques jours. Classiquement, les cellules détachées de leur support sont dénombrées avant d'être ensemencées à un certain ratio de cellules/cm² en boites de culture.

Les cellules souches de l'invention présentent également un profil de cycle cellulaire spécifique des cellules souches, à savoir moins de 35%, de préférence moins de 31% des cellules en phase G0/G1.

Préférentiellement, les caractéristiques phénotypiques des cellules selon l'invention sont plus particulièrement les suivantes :
▪ Morphologie de type petite cellule ronde ou ovoïde réfringente avec un rapport nucléocytoplasmique important
▪ Expression et activité télomérase
▪ Répartition des phases du cycle cellulaire particulière avec une partie des cellules en G0/G1 faible (< 35%) comparativement à des cellules somatiques (> 80%)
▪ Non expression des gènes endogènes de pluripotence comme OCT4, SOX2 et NANOG
▪ Expression de gènes GH1, LRP2, UGT8, JAG2, BRCA2, NBEA, CAMK2B, DSP, AMPH, SCIN, IGSF11, SLC6A15, CECR2 et WNK2.

Lorsque les cellules souche de l'invention sont obtenues par l'expression exogènes de CDX-2, c-MYC et OCT4, elles expriment en outre de préférence l'un au moins des gènes suivants: APLP2, BEX5, ADGRG2, B3GNT3, CIST1, KCNK17, MYH14, ADCY1. Plus préférentiellement, elles expriment au moins deux, au moins trois, au moins quatre, au moins cinq, au moins six, au moins sept ou huit de ces gènes. Encore plus préférentiellement, elles expriment l'ensemble de ces huit gènes.

Dans ce cadre, les gènes mentionnés sont les suivants :

**Tableau 3 : selon la base NCBI (https://www.ncbi.nlm.nih.gov/ ;rubrique de recherche GENE**

| **Gene *** | **Gene ID** | **NCBI SeqRef** | **Version** | **Nom du gène** |
|---|---|---|---|---|
| POU5F1 | 282316 | NM_174580.2 | 14-AVRIL-2016 | POU class 5 homeobox 1 |
| APLP2 | 785005 | XM_005197314.3 | 26-JAN-2016 | amyloid beta precursor like protein 2, transcript variant X3 |
| BEX5 | 516056 | NM_001075735.1 | 26-AOUT-2016 | brain expressed X-linked 5 |
| ADGRG2 | 100299135 | XM_015470573.1 | 26-JAN-2016 | adhesion G protein-coupled receptor G2, transcript variant X2 |
| B3GNT3 | 784997 | NM_001098473.1 | 24-AVR-2016 | UDP-GlcNAc:betaGal beta-1,3-N-acetylglucosaminyltransferase 3 |
| CIST1 | 506589 | NM_001075271.1 | 04-JAN-2015 | intestine-specific transcript 1 protein |
| KCNK17 | 282264 | NM_174558.2 | 18-AOUT-2016 | potassium channel, two pore domain subfamily K, member 17 |
| MYH14 | 514212 | XM_010823804.2 | 26-JAN-2016 | myosin, heavy chain 14, non-muscle, transcript variant X3 |
| ADCY1 | 281601 | NM_174229.2 | 18-AOUT-2016 | adenylate cyclase 1 (brain) |

Selon un troisième aspect, la présente invention concerne également l'utilisation de l'expression exogène des gènes CDX-2 et c-MYC dans des cellules somatiques de ruminants pour préparer des cellules souches.

De manière similaire à ce qui a été décrit ci-dessus en relation avec le procédé d'obtention de ces cellules souches, l'utilisation selon l'invention peut comprendre, outre l'expression des gènes CDX-2 et c-MYC, l'expression exogène additionnelle d'au moins un gène choisi dans le groupe constitué de OCT4, SOX2 et KLF4, de préférence au moins OCT4, dans lesdites cellules somatiques.

Tous les modes de réalisation préférées et toutes les précisions indiquées ci-dessus en relation avec la mise en oeuvre du procédé selon l'invention s'appliquent mutatis mutandis à l'aspect de l'invention concernant l'utilisation.

Enfin, la présente invention concerne également l'utilisation des cellules souches de l'invention pour l'expansion d'un fond génétique individuel, ou l'identification génomique de ce fond génétique. Les cellules souches de l'invention peuvent également être utilisées pour cribler des molécules anti-prolifératrices et tester l'action de molécules anti-proliférative, en particulier de certaines drogues utilisées pour le traitement de pathologies cancéreuses, mais aussi pour répliquer et tester la virulence de pathogènes chez les ruminants, notamment de souches pathogènes de tuberculose bovine, ou pour étudier les mécanismes d'infection et de propagation de ces pathogènes. Enfin, ces cellules peuvent aussi être utilisées dans le cadre d'un procédé de régénération vétérinaire avec une application toute particulière en reproduction animale du fait du potentiel de ces cellules pour la différenciation en cellules germinales.

### DESCRIPTION DES FIGURES

**Figure 1****.** Courbe de croissance des cellules BEF indiquant leur entrée en sénescence.
**Figure 2****.** Morphologie des cellules BCM (MC et OMC) comparativement aux cellules BEF avant reprogrammation
**Figure 3****.** Courbe de prolifération des cellules BCM (MC et OMC) et BEF
**Figure 4****.** Activité de la télomérase dans les cellules BCM (MC, OMC) et BEF
**Figure 5****.** Les cellules BCM prolifèrent dans des conditions non adhérentes
**Figure 6****.** Les cellules OMC expriment les protéines CDX2, c-MYC et OCT4 en l'absence (-Dox), en présence (+Dox) et après sevrage en doxycyline (Dox0)

### EXEMPLES

### Exemple 1. Courbe de croissance de fibroblastes primaires

Le potentiel prolifératif de fibroblastes embryonnaires a été évalué, ces données n'étant pas disponibles dans la littérature de façon surprenante. Des cultures primaires de fibroblastes embryonnaires bovins (BEF - *Bovine Embryonic Fibroblast)* ont été réalisées à partir de la mise en culture d'explants prélevés sur des foetus de 70 jours. Les cellules qui prolifèrent à partir de ces explants ont été dissociées et ré-ensemmencées à une concentration de 10³ à 2x10³ cellules par cm². Ces cellules présentent une morphologie typique de fibroblastes avec l'apparence d'une cellule allongée, plus ou moins aplatie, avec un cytoplasme abondant. Pour l'établissement de leur potentiel prolifératif à long terme, les cellules ont été dissociées à confluence en présence de trypsine selon un protocole classique d'entretien de fibroblastes et maintenues jusqu'à leur entrée en sénescence, phénomène observé par un ralentissement de leur potentiel prolifératif et un changement morphologique avec l'apparition de cellules géantes avec un cytoplasme qui conduit à l'arrêt progressif de la prolifération et à la disparition de la culture. A chaque passage, 1x10⁶ cellules dissociées ont été ensemencées en boite de culture de 55 cm² dans du milieu appelé milieu BEF composé d'un base DMEM/F12 additionné de 10% de sérum de veau foetal (FBS), de 1% L-glutamine (2mM), de 1000 U/mL de penicilline, 1000 U/ mL streptomycine et maintenues dans un incubateur à 38,5°C, à 7.5% de CO₂. Le milieu a été changé tous les 2-3 jours. Lorsque les cellules arrivent à confluence, elles ont été rincées au PBS et dissociées avec 0.05% de Trypsine-EDTA dont l'action a été stoppée par l'addition de milieu de culture complet. Après centrifugation à 400g, les cellules dissociées ont été reprises dans du milieu complet, comptées et réensemencées comme indiqué. Les courbes de croissance sont établies à partir des comptages.

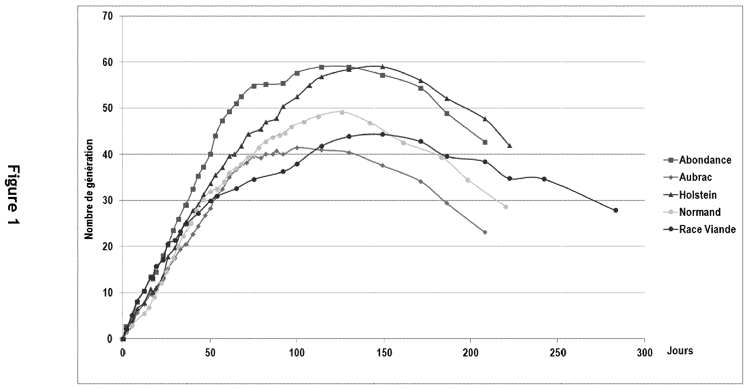

L'ensemble des différents prélèvements réalisés sur différentes races donnent des résultats similaires quant au potentiel de prolifération. Les courbes, représentées sur la Figure 1, indiquent une entrée en sénescence (nombre limité de générations) observée à partir de 40 à 60 générations selon les races correspondant au 15-20^{ème} passage quand le nombre de cellules récoltées devient identique ou même inférieur au nombre de cellules ensemencées après un temps de culture supérieur à la moyenne des passages précédents, environ 3-4 jours entre chaque dissociation pendant la période de prolifération active. Au cours de ce processus de prolifération et de sénescence, les cellules changent progressivement de morphologie et prennent une morphologie de cellules plus larges et aplaties avec un cytoplasme qui devient hétérogène.

Le différentiel de prolifération entre les fibroblastes et les cellules obtenues par reprogrammation peut ainsi être observé facilement.

### Exemple 2. Constructions de vecteurs transposons inductibles

Le génome bovin n'est que partiellement annoté surtout en comparaison du génome humain, dont la connaissance est beaucoup plus importante. En comparant les phases codantes des gènes OCT4, SOX2, KLF4, c-MYC, NANOG et CDX2 humains et bovins, il apparait que les identités sont très proches (Tableau 4). Les constructions ont donc été réalisées avec les phases codantes humaines.

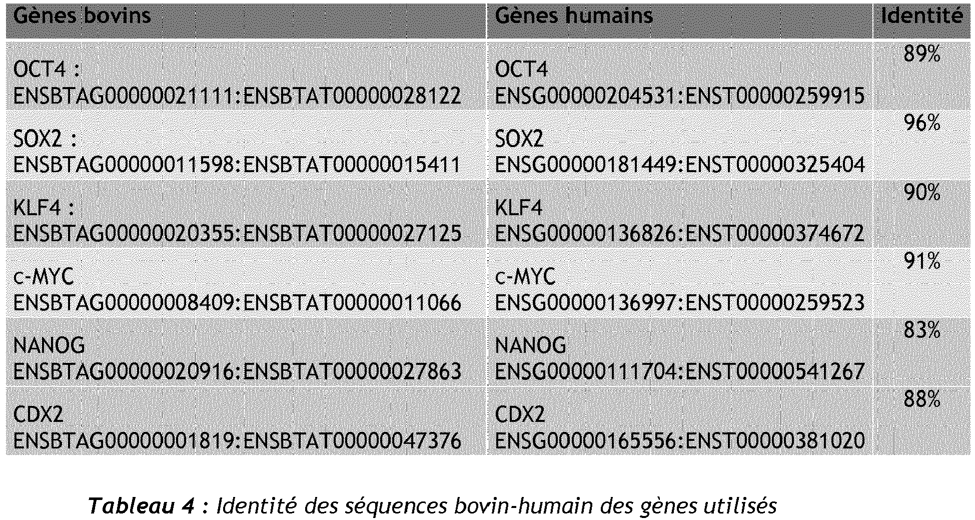

Les cDNAs humains ont été insérés dans les vecteurs pPB inductibles (Glover et al., 2013). L'ensemble des constructions ont été obtenues avec la technologie Gibson (NEB) à l'aide des oligonucléotides de clonage (Tableau 5) permettant le clonage des phases codantes.

L'insertion des phases codantes a été vérifiée par séquençage.

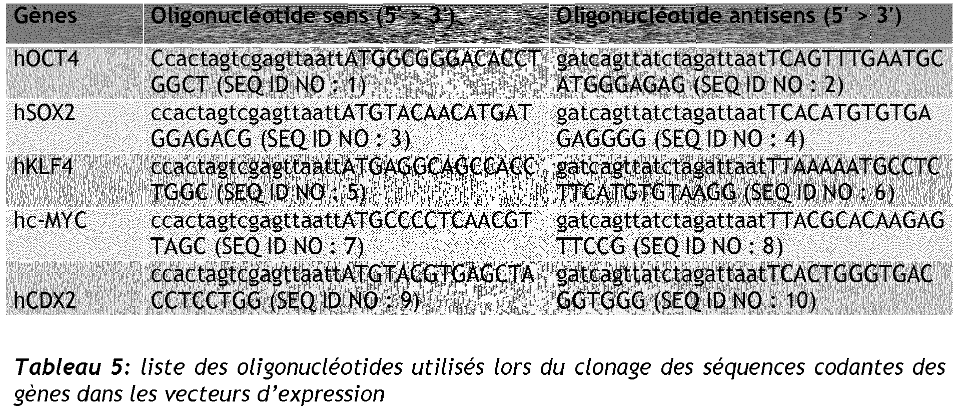

### Exemple 3. Le cocktail OSKM n'est pas suffisant pour établir une cellule reprogrammée en prolifération à long terme chez les bovins

Différents tests de reprogrammation ont été menés sur les BEF (voir Exemple 1) à des passages précoces (< passage 10). Dans les tests effectués avec les vecteurs transposons, les cellules BEF ont été modifiées par électroporation. Après dissociation, les cellules ont été centrifugées à 1200rpm (300g) à température ambiante pendant 5min, après aspiration du surnageant, le culot cellulaire a été rincé dans du PBS, centrifugé à nouveau et 1x10⁶ cellules directement reprises dans 120µL de tampon de resuspension (Neon, Life Technologies). Dans ce mélange a été ajouté sous forme de plasmide purifié, 2µg de transposase (1/3 de la quantité totale de plasmides) et 4µg de vecteurs (2/3 de la quantité totale de plasmides), mélange composé des différents transposons Piggybac inductibles à la doxycycline dont la composition varie selon les combinaisons testées. Le mélange cellules-plasmides a été électroporé par le système Neon (Life Technologies), à 1200V, pendant 40ms, en 1 pulse, dans un cône de 100µL, plongé dans une cuve contenant du tampon d'électroporation (Neon, Life Technologies). Après électroporation, les cellules ont été mises en culture dans un puits de plaque 6 puits, dans 3 mL de milieu complet pour fibroblastes.

Le milieu des cellules électroporées a été changé après 24h et une sélection par la puromycine à 1µg/mL et par la néomycine à 250µg/mL a été réalisée en fonction des gènes de résistance portés par les plasmides présents dans la combinatoire. Le milieu avec sélection a été changé tous les deux jours pendant au moins une semaine.

A la fin de la sélection entre 8 et 12 jours, les cellules ont été dissociées par la 0.05% trypsine-EDTA(Life) et 10⁵ cellules ont été ensemencées dans un puits de plaque de 6 puits dans 3 mL de milieu. Deux milieux de culture bien connus par l'homme du métier dans le domaine de la reprogrammation cellulaire ont été testés :
Le milieu « Stem » apparenté à un milieu 'EpiSC' comme défini dans le modèle souris est un milieu asérique composé de DMEM/F12 (250 mL), supplémenté de milieu Neurobasal (250 mL), des Suppléments B27 (5 mL), des suppléments N2 (2,5 mL), de 2mM L-Glutamine, de 1000 U/mL penicilline, 1000 U/mL streptomycine et de 1mM β-mercapto-éthanol. A ce milieu sont ajoutés 5ng/mL de b-FGF et 5ng/mL d'Activine A humaine (Peprotech), en présence de doxycycline à 2µg/mL final (Sigma).
Le milieu « ES » est composé de DMEM/F12 (500 mL) supplémenté de 10% FBS, de 2mM L-Glutamine, de 1000 U/mL penicilline, 1000 U/mL streptomycine et de 1mM β-mercapto-éthanol, de 100 nM Pyruvate de sodium, de 1% de Non essential Amino acid. Le facteur LIF (1000 U :mL), est ajouté en présence de doxycycline à 2µg/mL final.
Dans le milieu « Stem », des changements morphologiques apparaissent en moyenne à partir de 4 à 5 jours après l'addition de la doxycycline alors qu'il faut attendre au moins 7 à 10 jours pour le milieu « ES ». Ces changements sont dépendants de la présence de différentes phases codantes présentes dans les combinatoires.

Les colonies de cellules modifiées ont été prélevées après 10 - 12 jours d'induction et ensemencées dans des puits de plaque de 24 puits préalablement traités dans un mélange poly-L-ornithine - laminine (Sigma) permettant une meilleure adhésion. La prolifération et la morphologie des cellules est très différente de celle des fibroblastes BEF et des cellules non induites maintenues en absence de Doxycycline. Mais après un changement prolifératif et morphologique initial, les cellules modifiées reprennent progressivement une morphologie fibroblastique après plusieurs passages et ne peuvent pas s'établir dans le long terme. Cela est conforme aux observations publiées (Sumer et al., 2011).

### Exemple 4. Le gène CDX2 induit un changement en association avec c-MYC

Les cellules somatiques dérivées de biopsies embryonnaires (voir Exemple 1) ont été mises en culture jusqu'à leur confluence. Elles ont alors été dissociées et transduites pour permettre l'expression des vecteurs d'expression des transgènes inductibles dans le milieu BEF (voir Exemples 2 et 3).

Après la sélection des cellules modifiées, celles-ci sont alors mises dans le milieu Stem et induites en présence de Doxycyline. Les changements morphologiques interviennent dès le 4^{ème} ou 5^{ème} jour après le début de l'induction et se maintiennent de façon stable à partir de 8 à 10 jours après le début de l'induction. Ces cellules sont alors passées et amplifiées et gardent leurs caractères nouvellement acquis par ce processus.

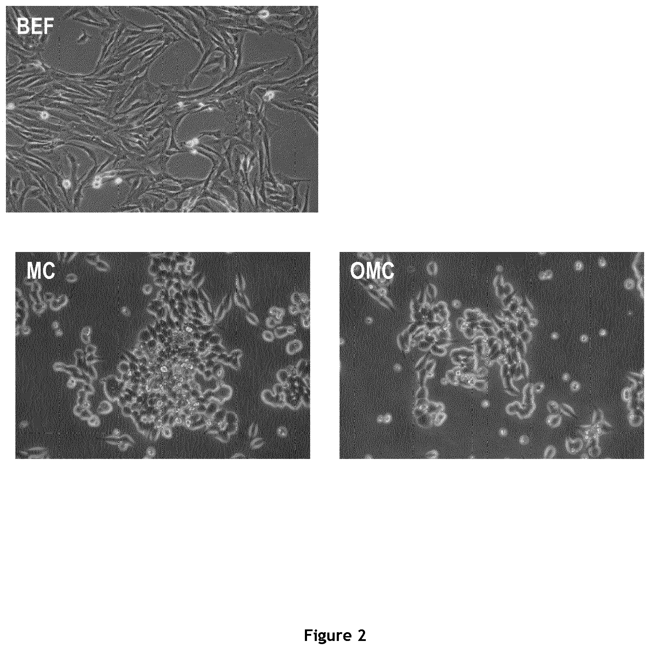

La seule expression de CDX2 dans les cellules BEF n'induit à elle seule aucun changement morphologique ou prolifératif notable comparativement à un BEF non modifié. De la même façon, la seule présence de c-MYC n'induit pas de changement majeur de morphologie et une prolifération à peine plus importante. Mais l'expression combinée de CDX2 et c-MYC induit un changement morphologique majeur des cellules, comme illustré sur la Figure 2. Les cellules qui expriment les gènes exogènes CDX2 et c-MYC (cellules MC) et les cellules qui expriment les gènes exogènes CDX2, c-MYC et OCT4 (cellules OMC) deviennent très petites, rondes ou ovoïdes et très réfringentes avec un rapport nucléocytoplasmique très élevé au contraire des cellules fibroblastes.

La présence d'autres gènes de pluripotence comme OCT4, SOX2 et KLF4 dans le cocktail de reprogrammation ne modifie pas de façon significative la cinétique d'apparition des colonies de cellules BCM, ni leur morphologie.

### Exemple 5. Les cellules BCM sont obtenues dans différentes races de ruminants

Des fibroblastes d'origine Abondance, Holstein ou race à viande ont été transduits dans les conditions décrites avec différentes combinaisons. Après sélection, le processus d'induction des transgènes a été déclenché par l'addition de Doxycyline dans le milieu de culture et les changements morphologiques ont été observés après 5 à 7 jours d'induction avec un délai un peu plus important et une ampleur un peu moindre pour la race à viande (Tableau 6).

Il n'y a pas de différence morphologique majeure dans les cellules obtenues.

**Tableau 6**

| | Race | | |
|---|---|---|---|
| | Abondance | Hollstein | Race à viande |
| | Obtention de cellules reprogrammées | | |
| Combinatoire | | | |
| C | - | - | - |
| M | - | - | - |
| MC | ++ | ++ | ++ |
| OMC | +++ | +++ | +++ |
| OSKM | - | - | - |

### Exemple 6. Les cellules BCM ont des caractéristiques de cellules souches

### Prolifération indéfinie

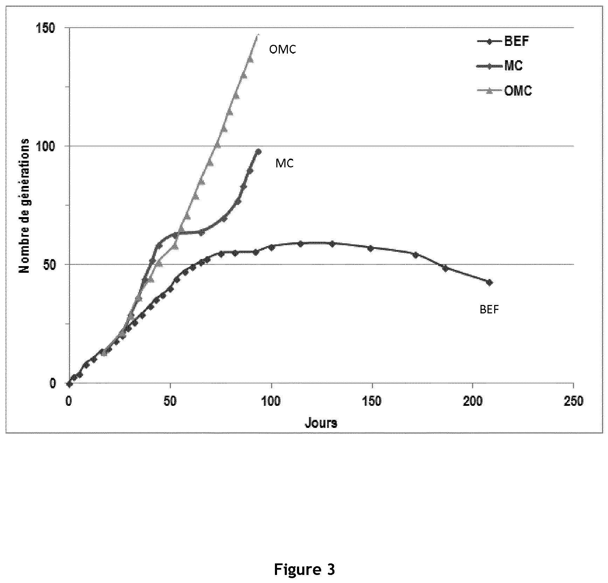

Les cellules BCM, obtenues dans un processus de reprogrammation impliquant au moins la combinatoire CDX2 et c-MYC ont été dissociées à chaque passage, comptées et réensemencées en présence de doxycycline et de facteurs. Selon les combinaisons utilisées (MC ou OMC), les courbes de prolifération permettent de calculer un temps de doublement moyen pour les seules cellules qui prolifèrent en absence d'inducteur (Figure 3). Il se situe entre 12,4 h et 15h pour les cellules BCM issues de la combinatoire MC (Tableau 7).

**Tableau 7 : Temps de doublement moyen des cellules obtenues dans différentes combinaisons**

| Cellules | Temps de doublement (h) |
|---|---|
| | |
| BEF | 60 |
| MC | 15 |
| OMC | 13,2 |

De plus, le potentiel prolifératif de ces cellules est aussi lié à l'absence de sénescence contrairement aux cellules BEF initiales.

### Activité télomérase

L'activité télomérase a été mesurée dans les cellules BCM en comparaison avec les BEF, en utilisant le kit de détection commercial TeloTAGGG Telomerase PCR ELISA (Roche).

Les cellules ont été lysées et l'activité de la télomérase a été évaluée par une amplification spécifique des répétitions télomériques (TRAP) suivie d'un d'ELISA comme recommandé par le fabriquant. Le contrôle positif (Ctrl+) est fourni par le fabriquant comme témoin d'une activité importante de la télomérase.

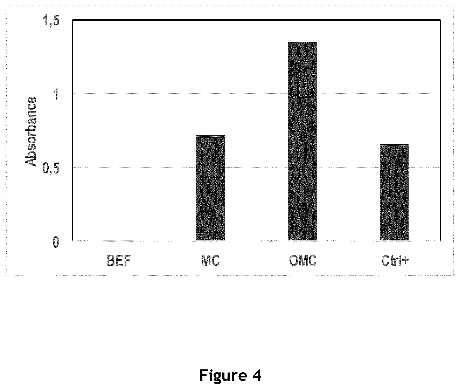

Les cellules BCM montrent une activité supérieure de 90 et 170 fois par rapport aux cellules négatives BEF, respectivement pour les cellules MC et les cellules OMC (Figure 4).

### Cycle cellulaire spécifique

Les cellules BCM ont également un profil de cycle cellulaire particulier. Des cellules en prolifération dans les milieux de routine ont été fixées et leur niveau moyen d'ADN a été évalué par coloration au iodure de propidium selon un protocole classique tel que décrit (Coronado et al ; 2013). Les cellules ont été dissociées, comptées, lavées 2 fois puis fixées à l'éthanol 70% à -20°C pendant 2h minimum. Le culot cellulaire a alors été resuspendu dans 5 mL de PBS et réhydraté par lavage au PBS plusieurs fois puis incubé 30 min dans du PBS additionné de 100 µg/mL de RNAse. Les cellules ont été analysées au FACS (Becton) après addition de 50 µg /mL de iodure de propidium par un laser Yellow Green (PE TexasRed). Les cycles ont été réalisés avec 50 000 évènements et analysés à l'aide du logiciel d'analyse FlowJo pour obtenir les pourcentages des différentes phases.

Le profil de cycle cellulaire des cellules OMC vs BEF est le suivant :

| | |
|---|---|
| % G0/G1 | 26,9 vs 83,9 |
| %S | 52,6 vs 6.9 |
| %G2 | 10.3 vs 6.5 |

Le profil de cycle cellulaire des cellules MC vs BEF est le suivant :

| | |
|---|---|
| % G0/G1 | 30,4 vs 83.9 |
| %S | 50 vs 6.9 |
| %G2 | 11,3 vs 6.5 |

Les données du cycle cellulaire observées pour les cellules MC et OMC, induites par le procédé de reprogrammation selon l'invention, sont typiques d'une cellule souche et en forte prolifération.

Les caractéristiques des cellules obtenues selon l'invention correspondent bien à des caractéristiques de cellules souches de par leur auto-renouvellement, leur activité télomérase, et leur profil de cycle cellulaire.

### Exemple 7. Les cellules BCM prolifèrent en condition non adhérente

A l'issue de l'obtention des cellules BCM, les cultures sont stabilisées par la présence d'un traitement dans le puits de culture qui permet de les maintenir, de les amplifier et de les propager. Les cellules BCM prolifèrent sous une forme adhérente dans des puits traités à la poly-ornithine laminine et sont préférentiellement non adhérente en absence de traitement ou même quand les puits sont traités par la gélatine ou la fibronectine.

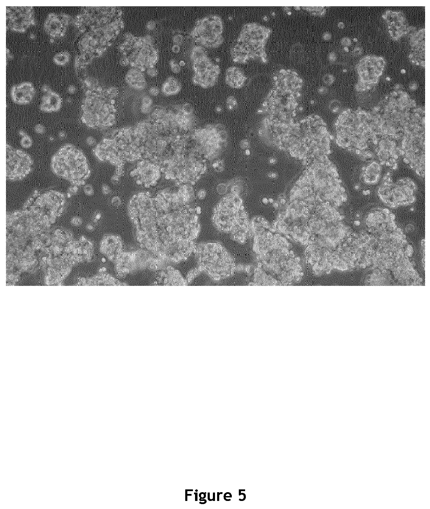

Ces cellules peuvent se maintenir sous forme de petits agrégats non adhérents en prolifération dans des flacons de culture pour cellules non adhérentes (Figure 5).

Les cellules BCM de l'invention sont donc capables de proliférer aussi bien en condition adhérentes (Figure 2) que non adhérentes (Figure 5).

### Exemple 8. Les cellules BCM expriment les protéines c-MYC, OCT4 et CDX2 exogènes

Par des analyses de Western blot, il est possible d'étudier l'expression des protéines exogènes OCT4, CDX2 et c-MYC avec des anticorps dirigés contre les protéines humaines.

Pour cela, les cellules ont été lysées dans un tampon RIPA (150mM sodium chloride, 1% Triton X-100, 0.5% sodium deoxycholate, 0.1% SDS, 50mM Tris, pH 8, et protease inhibitor cocktail), incubées 30min dans la glace, et centrifugées. Le surnageant contenant les protéines a été dosé (Coomassie Plus Bradford Assay Kit) à 595nm. 10µg de protéines ont été déposées et mis à migrer sur gel d'acrylamide 12% dans un tampon Trizma 250mM, Glycine 1900mM et SDS 1%, pendant 1h30 à 100V, en présence d'un marqueur de taille (Biorad Precision Plus Protein WesternC Standards #161-0376). Les protéines ont été transférées sur membrane de nitrocellulose en tampon Trizma 25mM, Glycine 190mM et méthanol 20% pendant 1h à 100V. La membrane a ensuite été incubée en tampon de blocage (TBS-Tween 5% de lait) pendant 1h puis avec un des anticorps suivants, en tampon de blocage, toute la nuit à 4°C ou 1h à température ambiante :

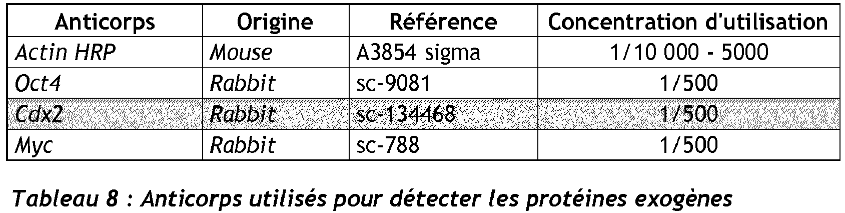

La membrane a ensuite été lavée 4 fois 5min en TBS-Tween puis incubée avec l'anticorps secondaire, en tampon de blocage 1h à température ambiante : Anti-rabbit HRP NA 934VS ECL Peroxidase 1 /10 000. Après 4 lavages, les protéines ont été révélées grâce au Clarity ECL Substrate.

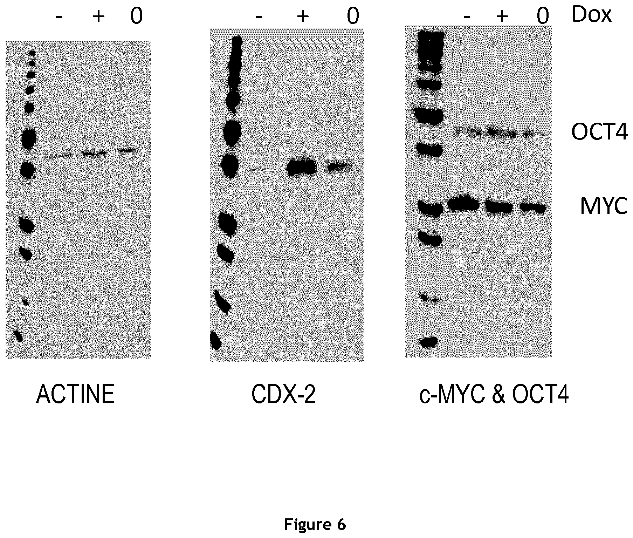

Comme illustré sur la Figure 6, les cellules OMC obtenues à l'aide de la combinatoire CDX2, c-MYC et OCT4 expriment bien les protéines exogènes correspondantes en présence de doxycycline (+Dox) et maintiennent leur expression après sevrage des cellules en doxycyline (Dox0).

### Exemple 9. La combinatoire minimale CDX2/c-MYC induit une reprogrammation dans d'autres espèces que le bovin

Afin de tester l'effet du gène CDX2 et de son association avec c-MYC dans la combinatoire, la reprogrammation a été testée sur les fibroblastes embryonnaires de mammifères, cellules dérivées d'embryons de bovin de 70 jours, de fibroblastes d'embryon de 30 à 740 jours de mouton et de chèvre.

Toutes les cellules ont été transduites par les vecteurs d'expression avec les phases codantes humaines (bovin, mouton, chèvre).

Les changements morphologiques importants ont été observés pour les cellules de bovin, de mouton, et de chèvre avec la possibilité d'obtenir des cellules qui s'établissent et présentent des morphologies très similaires aux cellules BCM (Tableau 9, le symbole « + » est en correspondance avec l'importance des changements morphologiques correspondants à ceux de cellules souches).

Aucun changement notable n'est observé avec les cellules murines ni aviaires.

**Tableau 9 : Modifications morphologiques observées avec les gènes CDX2 et c-MYC seuls ou en combinaison, éventuellement combinés à OCT4.**

| **Combinatoire** | **BEF** | **SEF** | **GEF** |
|---|---|---|---|
| C | - | - | - |
| M | - | - | - |
| MC | ++ | ++ | ++ |
| OMC | ++++ | ++++ | ++++ |

### Exemple 10. Analyse de l'ARN des cellules BCM

Une analyse de l'ARN seq a été effectuée sur les cellules BEF, MC et OMC. 1 µg de RNA total extrait des cellules a été utilisé pour préparer la banque en utilisant le kit de préparation d'échantillons d'ARNm TruSeq R Stranded (Illumina) selon la recommandation du fabricant. La taille moyenne des fragments, estimée par Bioanalyser (Agilent) étaient de 281 and 280 bp pour les deux échantillons de BEF, de 280 et 282 pour les 2 échantillons de cellules MC et de 281 pour les cellules OMC. La molarité, estimée par Qubit (Life Technologies) était de 250,19 et 253,79 nM pour les BEF, de 243,93 et 256,56 nM pour les cellules MC et de 236,71 nM pour les cellules OMC. Chaque librairie est diluée à 10 nM dans du Tris-HCl 10 mM, 0,1% Tween 20. Les échantillons sont mélangés, dilués à 4 nM, dénaturés dans une solution de NaOH 0,2 N à température ambiante pendant 5 min puis neutralisés par l'addition de TrisHCl 200 mM ph 7. Une dilution à une concentration de 20pM est préparée dans du tampon d'hybridation. Le séquençage en double lecture 'paired end sequencing' est réalisé sur le séquenceur NextSeq 500 (Illumina) à l'aide du kit 'nextSeq500 high output v2 (illumina) et la qualité du séquençage contrôlée par le logiciel de visualisation Sequencing Analysis Viewer 1.8.37 (Illumina). Le contrôle qualité des fichiers 'Sample-ID.fastq' est effectué avec le logiciel FasQC (Babraham Institute). Le nombre total de lectures était de 54362226 et 587473448 pour les BEF, de 60 075 319 et 37 028 949 pour les cellules MC et de 90 334 233 pour les cellules OMC. L'alignement des séquences a été réalisé sur le génome du bovin, version Btau 5.0.1 - https://www.ncbi.nlm.nih.gov/assembly/GCA_000003205.6) à l'aide du paquet Hisat, subread et R (DEseq2) (Love et al., 2014).

Parmi les gènes les plus différentiellemnt exprimés entre les cellules BEF et les cellules BCM (MC et OMC), on trouve notamment, outre CDX2 et le cas échéant OCT4 comme gènes exogènes, les gènes GH1, LRP2, UGT8, JAG2, BRCA2, NBEA, CAMK2B, DSP, AMPH, SCIN, IGSF11, SLC6A15, CECR2, WNK2. Les résultats sont regroupés dans le Tableau 10 ci-dessous.

**Tableau 10: selon la base NCBI (https://www.ncbi.nlm.nih.gov/ ;rubrique de recherche GENE**

| **Gene *** | **Gene ID** | **NCBI SeqRef** | **Version** | **"Fold change" MC vs BEF** | **"Fold change" OMC vs BEF** | **Nom du gène** |
|---|---|---|---|---|---|---|
| CDX2 | 618679 | NM_001206299.1 | 24-DEC-2015 | 11,45 | 10,63 | caudal type homeobox 2 |
| GH1 | 280804 | NM_180996.1 | 24-AVR-2016 | 11,21 | 10,45 | growth hormone 1 |
| LRP2 | 100337021 | XM_015462263.1 | 26-JAN-2016 | 10,55 | 9,61 | LDL receptor related protein 2 |
| UGT8 | 281566 | NM_001083635.1 | 24-AVR-2016 | 10,54 | 10,39 | UDP glycosyltransferase 8 |
| JAG2 | 517405 | XM_015468638.1 | 26-JAN-2016 | 10,13 | 10,40 | jagged 2, transcript variant X1 |
| BRCA2 | 507069 | XM_002684277.4 | 26-JAN-2016 | 10,08 | 9,29 | breast cancer 2, transcript variant X1 |
| NBEA | 525624 | XM_015465645.1 | 26-JAN-2016 | 10,00 | 9,41 | neurobeachin, transcript variant X5 |
| CAMK2B | 525416 | NM_001035357.2 | 29-JUIL-2016 | 9,79 | 10,26 | calcium/calmodulin-dependent protein kinase II beta |
| DSP | 514360 | NM_001192368.1 | 04-JAN-2015 | 9,65 | 10,12 | desmoplakin |
| AMPH | 614722 | NM_001098130.1 | 24-AVR-2016 | 9,57 | 9,15 | amphiphysin |
| SCIN | 281478 | NM_174177.2 | 24-AVR-2016 | 9,38 | 9,26 | scinderin |
| IGSF11 | 540003 | NM_001076921.1 | 25-AOUT-2016 | 9,22 | 8,99 | immunoglobulin superfamily member 11 |
| SLC6A15 | 281952 | NM_181023.2 | 25-AOUT-2016 | 9,18 | 7,87 | solute carrier family 6 (neutral amino acid transporter), member 15 |
| CECR2 | 517202 | XM_015463582.1 | 26-JAN-2016 | 9,07 | 8,45 | cat eye syndrome chromosome region, candidate 2 |
| WNK2 | 506520 | XM_005199274.3 | 26-JAN-2016 | 9,03 | 8,53 | WNK lysine deficient protein kinase 2, transcript variant X3 |

Parmi les gènes différentiellement exprimés entre les cellules MC et OMC, on trouve notamment, outre POU5F1 (= OCT4) comme gène exogène, les gènes APLP2, BEX5, ADGRG2, B3GNT3, CIST1, KCNK17, MYH14, ADCY1. Les résultats sont regroupés dans le Tableau 11 ci-dessous.

**Tableau 11: selon la base NCBI (https://www.ncbi.nlm.nih.gov/ ;rubrique de recherche GENE**

| **Gene *** | **Gene ID** | **NCBI SeqRef** | **Version** | **"Fold change" MC vs OMC** | **Nom du gène** |
|---|---|---|---|---|---|
| POU5F1 | 282316 | NM_174580.2 | 14-AVRIL-2016 | -10,47 | POU class 5 homeobox 1 |
| APLP2 | 785005 | XM_005197314.3 | 26-JAN-2016 | -5,78 | amyloid beta precursor like protein 2, transcript variant X3 |
| BEX5 | 516056 | NM_001075735.1 | 26-AOUT-2016 | -5,30 | brain expressed X-linked 5 |
| ADGRG2 | 100299135 | XM_015470573.1 | 26-JAN-2016 | -5,01 | adhésion G protein-coupled receptor G2, transcript variant X2 |
| B3GNT3 | 784997 | NM_001098473.1 | 24-AVR-2016 | -4,93 | UDP-GlcNAc:betaGal beta-1,3-N-acetylglucosaminyltransferase 3 |
| CIST1 | 506589 | NM_001075271.1 | 04-JAN-2015 | -4,24 | intestine-specific transcript 1 protein |
| KCNK17 | 282264 | NM_174558.2 | 18-AOUT-2016 | -4,08 | potassium channel, two pore domain subfamily K, member 17 |
| MYH14 | 514212 | XM_010823804.2 | 26-JAN-2016 | -3,95 | myosin, heavy chain 14, non-muscle, transcript variant X3 |
| ADCY1 | 281601 | NM_174229.2 | 18-AOUT-2016 | -3,86 | adenylate cyclase 1 (brain) |

### Conclusions

Le gène CDX2 présente un potentiel de reprogrammation et permet dans une combinaison minimale avec c-MYC d'obtenir un nouveau type cellulaire, les cellules BCM. Ces cellules BCM prolifèrent rapidement, indéfiniment, ont une activité télomérase et un cycle cellulaire spécifique correspondant, et expriment différents marqueurs propres aux cellules souches.

Cette combinatoire minimale CDX2 / c-MYC fonctionne aussi dans des cellules de mouton et de chèvre. Les cellules souches obtenues présentent des morphologies très similaires dans toutes les espèces.

### REFERENCES

Anokye-Danso F, Trivedi CM, Juhr D, Gupta M, Cui Z, Tian Y, Zhang Y, Yang W, Gruber PJ, Epstein JA, Morrisey EE. (2011). Highly efficient miRNA-mediated reprogramming of mouse and human somatic cells to pluripotency. Cell Stem Cell. 8: 376-88.
Chambers I, Tomlinson SR. (2009). The transcriptional foundation of pluripotency. Development 136: 2311-22.
Coronado D, Godet M, Bourillot PY, Tapponnier Y, Bernat A, Petit M, Afanassieff M, Markossian S, Malashicheva A, lacone R, Anastassiadis K, Savatier P. (2013). A short G1 phase is an intrinsic determinant of naïve embryonic stem cell pluripotency. Stem Cell Res. 10: 118-31.
De Los Angeles A, Ferrari F, Xi R, Fujiwara Y, Benvenisty N, Deng H, Hochedlinger K, Jaenisch R, Lee S, Leitch HG, Lensch MW, Lujan E, Pei D, Rossant J, Wernig M, Park PJ, Daley GQ. (2015). Hallmarks of pluripotency. Nature.525: 469-78.
Declercq J1, Sheshadri P, Verfaillie CM, Kumar A. (2013); Zic3 enhances the generation of mouse induced pluripotent stem cells. Stem Cells Dev. 22: 2017-25.
Ezashi T, Telugu BP, Alexenko AP, Sachdev S, Sinha S, Roberts RM. (2009). Derivation of induced pluripotent stem cells from pig somatic cells. Proc Natl Acad Sci USA. 106: 10993-8.
Feng B, Jiang J, Kraus P, Ng JH, Heng JC, Chan YS, Yaw LP, Zhang W, Loh YH, Han J, Vega VB, Cacheux-Rataboul V, Lim B, Lufkin T, Ng HH. (2009); Reprogramming of fibroblasts into induced pluripotent stem cells with orphan nuclear receptor Esrrb. Nat Cell Biol. 11: 197-203.
Glover JD, Taylor L, Sherman A, Zeiger-Poli C, Sang HM, McGrew MJ. (2013). A novel piggyBac transposon inducible expression system identifies a role for AKT signalling in primordial germ cell migration. PLoS One. 8: e77222.
Han J1, Yuan P, Yang H, Zhang J, Soh BS, Li P, Lim SL, Cao S, Tay J, Orlov YL, Lufkin T, Ng HH, Tam WL, Lim B. (2010). Tbx3 improves the germ-line competency of induced pluripotent stem cells. Nature. 463(7284):1096-100.
Heng JC, Feng B, Han J, Jiang J, Kraus P, Ng JH, Orlov YL, Huss M, Yang L, Lufkin T, Lim B, Ng HH. (2010). The nuclear receptor Nr5a2 can replace Oct4 in the reprogramming of murine somatic cells to pluripotent cells. Cell Stem Cell. 6: 167-74.
Hochedlinger K, Plath K. (2009). Epigenetic reprogramming and induced pluripotency. Development. 136: 509-23. Review.
Honda A, Hatori M, Hirose M, Honda C, Izu H, Inoue K, Hirasawa R, Matoba S, Togayachi S, Miyoshi H, Ogura A. (2013). Naive-like conversion overcomes the limited differentiation capacity of induced pluripotent stem cells. J Biol Chem. 288: 26157-66
Hong Y, Winkler C, Liu T, Chai G, Schartl M. (2004). Activation of the mouse Oct4 promoter in medaka embryonic stem cells and its use for ablation of spontaneous differentiation. Mech Dev 121: 933-43.
Huang P1, Zhang L1, Gao Y1, He Z2, Yao D3, Wu Z3, Cen J1, Chen X1, Liu C2, Hu Y2, Lai D4, Hu Z5, Chen L6, Zhang Y6, Cheng X1, Ma X6, Pan G3, Wang X7, Hui L. (2014). Direct reprogramming of human fibroblasts to functional and expandable hepatocytes. Cell Stem Cell. 14: 370-84.
leda M, Fu JD, Delgado-Olguin P, Vedantham V, Hayashi Y, Bruneau BG, Srivastava D. (2010). Direct reprogramming of fibroblasts into functional cardiomyocytes by defined factors. Cell. 142: 375-86.
Kaji K, Norrby K, Paca A, Mileikovsky M, Mohseni P, Woltjen K. (2009). Virus-free induction of pluripotency and subsequent excision of reprogramming factors. Nature. 458: 771-5.
Koh S, Piedrahita JA. (2014). From "ES-like" cells to induced pluripotent stem cells: a historical perspective in domestic animals. Theriogenology. 2014 Jan 1;81(1):103-11. Liu J, Balehosur D, Murray B, Kelly JM, Sumer H, Verma PJ. (2012). Generation and characterization of reprogrammed sheep induced pluripotent stem cells. Theriogenology. 77: 338-46.
M. I. Love, W. Huber, S. Anders (2014). Moderated estimation of fold change and dispersion for RNA-seq data with DESeq2. Genome Biology 15 ; 550. http://dx.doi.org/10.1186/s13059-014-0550-8
Lu Y, West FD, Jordan BJ, Mumaw JL, Jordan ET, Gallegos-Cardenas A, Beckstead RB, Stice SL. (2012). Avian-induced pluripotent stem cells derived using human reprogramming factors. Stem Cells Dev. 21: 394-403.
Nichols J, Smith A. (2009). Naive and primed pluripotent states. Cell Stem Cell. 4: 487-92. Niwa H. (2007). How is pluripotency determined and maintained? Development. 134: 635-46.
Osteil P, Tapponnier Y, Markossian S, Godet M, Schmaltz-Panneau B, Jouneau L, Cabau C, Joly T, Blachère T, Gócza E, Bernat A, Yerle M, Acloque H, Hidot S, Bosze Z, Duranthon V, Savatier P, Afanassieff M. (2013). Induced pluripotent stem cells derived from rabbits exhibit some characteristics of naïve pluripotency. Biol Open. 2: 613-28.
Pain B, Clark ME, Shen M, Nakazawa H, Sakurai M, Samarut J, Etches RJ. (1996). Long-term in vitro culture and characterisation of avian embryonic stem cells with multiple morphogenetic potentialities. Development. 122: 2339-48.
Rosselló RA, Chen CC, Dai R, Howard JT, Hochgeschwender U, Jarvis ED. (2013); Mammalian genes induce partially reprogrammed pluripotent stem cells in non-mammalian vertebrate and invertebrate species. Elife.2:e00036.
Shimada H, Nakada A, Hashimoto Y, Shigeno K, Shionoya Y, Nakamura T. (2010). Generation of canine induced pluripotent stem cells by retroviral transduction and chemical inhibitors. Mol Reprod Dev. 77: 2.
Stadtfeld M, Hochedlinger K. (2010). Induced pluripotency: history, mechanisms, and applications. Genes Dev. 24: 2239-63. Review.
Sumer H, Liu J, Malaver-Ortega LF, Lim ML, Khodadadi K, Verma PJ. (2011); NANOG is a key factor for induction of pluripotency in bovine adult fibroblasts. J Anim Sci. 89: 2708-16.
Takahashi K, Yamanaka S. (2006). Induction of pluripotent stem cells from mouse embryonic and adult fibroblast cultures by defined factors. Cell. 126: 663-76.
Takahashi K1, Yamanaka S. (2013). Induced pluripotent stem cells in medicine and biology. Development. 140: 2457-61.
Tesar PJ, Chenoweth JG, Brook FA, Davies TJ, Evans EP, Mack DL, Gardner RL, McKay RD. (2007). New cell lines from mouse epiblast share defining features with human embryonic stem cells. Nature. 448: 196-9.
Verma R, Liu J, Holland MK, Temple-Smith P, Williamson M, Verma PJ. (2013). Nanog is an essential factor for induction of pluripotency in somatic cells from endangered felids. Biores Open Access. 2: 72-6.
Vierbuchen T, Ostermeier A, Pang ZP, Kokubu Y, Südhof TC, Wernig M. (2010). Direct conversion of fibroblasts to functional neurons by defined factors. Nature. 463: 1035-41.
Woltjen K, Michael IP, Mohseni P, Desai R, Mileikovsky M, Hämäläinen R, Cowling R, Wang W, Liu P, Gertsenstein M, Kaji K, Sung HK, Nagy A. (2009). piggyBac transposition reprograms fibroblasts to induced pluripotent stem cells. Nature. 458: 766-70.
Yang N, Zuchero JB, Ahlenius H, Marro S, Ng YH, Vierbuchen T, Hawkins JS, Geissler R, Barres BA, Wernig M. (2013). Generation of oligodendroglial cells by direct lineage conversion. Nat Biotechnol. 31: 434-9.
Yu B1, He ZY, You P, Han QW, Xiang D, Chen F, Wang MJ, Liu CC, Lin XW, Borjigin U, Zi XY, Li JX, Zhu HY, Li WL, Han CS, Wangensteen KJ, Shi Y, Hui LJ, Wang X, Hu YP. Reprogramming fibroblasts into bipotential hepatic stem cells by defined factors. (2013). Cell Stem Cell. 13: 328-40.
Yu J1, Vodyanik MA, Smuga-Otto K, Antosiewicz-Bourget J, Frane JL, Tian S, Nie J, Jonsdottir GA, Ruotti V, Stewart R, Slukvin II, Thomson JA. (2007); Induced pluripotent stem cell lines derived from human somatic cells. Science. 318: 1917-20.

## Revendications

1. Procédé de préparation *in vitro* de cellules souches, comprenant la culture de cellules somatiques de ruminants et l'expression exogène des gènes CDX-2 et c-MYC dans lesdites cellules somatiques.

2. Procédé de préparation de cellules selon la revendication 1, comprenant en outre l'expression exogène d'au moins un des gènes choisis dans le groupe constitué de OCT4, SOX2 et KLF4, de préférence au moins OCT4, dans lesdites cellules somatiques.

3. Procédé de préparation de cellules selon l'une quelconque des revendications précédentes, dans lequel l'expression exogène des gènes CDX-2 et c-MYC est réalisée pendant au moins 10 jours.

4. Procédé de préparation de cellules selon l'une quelconque des revendications précédentes, dans lequel l'expression desdits gènes est une expression inductible.

5. Procédé de préparation de cellules selon l'une quelconque des revendications précédentes, dans lequel lesdites cellules somatiques sont des cellules somatiques bovines, caprines ou ovines.

6. Procédé de préparation de cellules selon l'une quelconque des revendications précédentes, dans lequel lesdites cellules somatiques sont choisies dans le groupe constitué de fibroblastes, de cellules musculaires, les cellules blanches du sang circulant, les adipocytes, les hépatocytes et les cellules endothéliales.

7. Cellules souches de ruminants, susceptible d'être obtenues par le procédé selon l'une quelconque des revendications 1 à 6.

8. Cellules souches de ruminants selon la revendication 7, **caractérisées en ce qu'**elles expriment le gène de la télomérase TERT et l'un au moins des gènes suivants: GH1, LRP2, UGT8, JAG2, BRCA2, NBEA, CAMK2B, DSP, AMPH, SCIN, IGSF11, SLC6A15, CECR2, WNK2.

9. Cellules souches de ruminants selon la revendication 8, **caractérisées en ce qu'**elles présentent une activité télomérase.

10. Cellules souches de ruminants selon la revendication 8 ou la revendication 9, **caractérisées en ce qu'**elles ont un profil de cycle cellulaire avec moins de 35% des cellules en phase G0/G1.

11. Utilisation de l'expression exogène des gènes CDX-2 et c-MYC dans des cellules somatiques de ruminants pour préparer des cellules souches.

12. Utilisation selon la revendication 11, **caractérisée par** l'expression exogène additionnelle d'au moins un gène choisi dans le groupe constitué de OCT4, SOX2 et KLF4, de préférence au moins OCT4, dans lesdites cellules somatiques.

13. Utilisation des cellules souches selon l'une quelconque des revendications 7 à 10 pour l'expansion d'un fond génétique individuel ou l'identification génomique de ce fond génétique, pour cribler des molécules anti-prolifératrices et tester l'action de molécules anti-proliférative, pour répliquer et tester la virulence de pathogènes chez les ruminants et étudier les mécanismes d'infection et de propagation de ces pathogènes.


SEQUENCE LISTING

<110>  INSTITUT NATIONAL DE LA RECHERCHE AGRONOMIQUE
       ALLICE
       INSTITUT NATIONAL DE LA SANTE ET DE LA RECHERCHE MEDICALE
       (INSERM)

<120>  PROCEDE DE REPROGRAMMATION DE CELLULES SOMATIQUES DE RUMINANTS

<130>  B72949

<160>  10

<170>  PatentIn version 3.5

<210>  1
<211>  36
<212>  DNA
<213>  Artificial Sequence

<220>
<223>  primer sens hOCT4

<400>  1
ccactagtcg agttaattat ggcgggacac ctggct                                36


<210>  2
<211>  42
<212>  DNA
<213>  Artificial Sequence

<220>
<223>  primer antisens hOCT4

<400>  2
gatcagttat ctagattaat tcagtttgaa tgcatgggag ag                         42


<210>  3
<211>  39
<212>  DNA
<213>  Artificial Sequence

<220>
<223>  Primer sens hSOX2

<400>  3
ccactagtcg agttaattat gtacaacatg atggagacg                             39


<210>  4
<211>  38
<212>  DNA
<213>  Artificial Sequence

<220>
<223>  Primer antisens hSOX2

<400>  4
gatcagttat ctagattaat tcacatgtgt gagagggg                              38

<210>  5
<211>  36
<212>  DNA
<213>  Artificial Sequence

<220>
<223>  Primer sens hKLF4

<400>  5
ccactagtcg agttaattat gaggcagcca cctggc                                36


<210>  6
<211>  46
<212>  DNA
<213>  Artificial Sequence

<220>
<223>  Primer antisens hKLF4

<400>  6
gatcagttat ctagattaat ttaaaaatgc ctcttcatgt gtaagg                     46


<210>  7
<211>  36
<212>  DNA
<213>  Artificial Sequence

<220>
<223>  Primer sens hc-MYC

<400>  7
ccactagtcg agttaattat gcccctcaac gttagc                                36


<210>  8
<211>  38
<212>  DNA
<213>  Artificial Sequence

<220>
<223>  Primer antisens hc-MYC

<400>  8
gatcagttat ctagattaat ttacgcacaa gagttccg                              38


<210>  9
<211>  40
<212>  DNA
<213>  Artificial Sequence

<220>
<223>  Primer sens hCDX2

<400>  9
ccactagtcg agttaattat gtacgtgagc tacctcctgg                            40


<210>  10
<211>  38
<212>  DNA

<213>  Artificial Sequence

<220>
<223>  Primer antisens hCDX2

<400>  10
gatcagttat ctagattaat tcactgggtg acggtggg                              38